# EUROPEAN PATENT APPLICATION

(11) **EP 1 382 602 A1**
(43) Date of publication of application: **21.01.2004**
(21) Application number: 02718609.7
(22) Date of filing: 18.04.2002
(51) Int. Cl.: C07D 301/19, C07D 303/04

(54) **PROCESS FOR PRODUCING PROPYLENE OXIDE**

(30) Priority: 27.04.2001 JP 2001132003
(71) Applicant: Sumitomo Chemical Company, Limited, Osaka-shi Osaka 541-8550 (JP)
(72) Inventor: OKU, Noriaki, Ichihara-shi, Chiba 290-0038 (JP); TSUJI, Junpei, Ichihara-shi, Chiba 299-0125 (JP)
(74) Representative: Henkel, Feiler, Hänzel
(86) International application number: PCT/JP2002/003849
(87) International publication number: WO 2002/088104

(57) **Abstract**

A process for producing propylene oxide, which comprises reacting propylene with cumene hydroperoxide in the presence of a catalyst, wherein a concentration of water contained in the propylene as a raw material is 2% by weight or less.

## Description

### TECHNICAL FIELD

The present invention relates to a process for producing propylene oxide. More particularly, the present invention relates to a process for producing propylene oxide, which converts propylene into propylene oxide using cumene hydroperoxide as an oxygen carrier, and further can be conducted under high yield.

### BACKGROUND ART

A process in which propylene is converted into propylene oxide using an organic peroxide as an oxygen carrier is publicly known, for example, in a case that ethylbenzene hydroperoxide is used as the organic peroxide to give propylene oxide and styrene, it is publicly known as Halcon process, and propylene oxide and styrene are produced. Further, when cumene hydroperoxide is used, cumyl alcohol together with propylene oxide is produced. Cumyl alcohol is converted into α-methylstyrene by dehydrogenation, or after cumyl alcohol is converted into cumene by hydrogenation, cumene can be used repeatedly by converting into cumene hydroperoxide again by oxidation.

Though a concept of a process in which only propylene oxide is produced using cumene repeatedly, is described in Czechoslovak Patent No. CS 140,743, the process described in said patent does not contain precise descriptions concerning necessary steps except oxidation step, epoxidation step and hydrogenolysis step. Various problems arise in practical recycling of cumene and therefore the patent cannot be said as sufficient for industrial realization.

### DISCLOSURE OF THE INVENTION

An object of the present invention is to provide a process for producing propylene oxide, which converts propylene into propylene oxide using cumene hydroperoxide as an oxygen carrier, and further can be conducted under high yield.

Namely, the present invention relates to a process for producing propylene oxide, which comprises reacting propylene with cumene hydroperoxide in the presence of a catalyst, wherein the propylene as a raw material has a water content of 2% by weight or less.

### BEST MODE FOR CARRYING OUT THE INVENTION

In the present invention, the epoxidation reaction means a reaction producing propylene oxide by reacting propylene with cumene hydroperoxide in the presence of a catalyst.

In the present invention, propylene oxide is obtained by subjecting cumene hydroperoxide obtained by auto-oxidation of cumene with air or air in which oxygen is concentrated, with propylene in the presence of a catalyst to the epoxidation reaction.

The epoxidation is preferably conducted in the presence of a catalyst containing a titanium-containing silicon oxide from the viewpoint that the desired product should be obtained under high yield and high selectivity. The catalyst is preferably a catalystcontainingtitanium chemically bonded to silicon oxide, so-called a titanium-silica catalyst. Examples may include products carrying a titanium compound on a silica carrier, products in which a titanium compound is compounded with a silicon oxide by a co-precipitation or sol-gel method, titanium-containing zeolite compounds and the like.

In the present invention, cumene hydroperoxide used as the raw material for the reaction step may be a dilute or thick purification or non-purification product.

The epoxidation reaction in the present invention can be conducted in a liquid phase using a solvent. The solvent is a liquid under the reaction temperature and pressure, and preferably substantially inert to the reactants and the product. The solvent may be composed of a substance existing in a solution of the hydroperoxide used. When, for example, a cumene solution of cumene hydroperoxide is used as a raw material, it is also possible to use cumene as a solvent without adding a solvent in particular. Other useful solvents include aromatic single-ring compounds (for example, benzene, toluene, chlorobenzene and o-dichlorobenzene), alkane (for example, octane, decane and dodecane) and the like. The reaction temperature in the epoxidation step is usually 0 to 200°C and preferably 25 to 200°C. The pressure is usually 100 to 20000 kPa, and preferably 100 to 10000 kPa taking account of the reaction temperature and economy.

The epoxidation step in the present invention can advantageously be carried out using a catalyst in the form of a slurry or a fixed-bed. The fixed-bed is preferred in the case of a large-scale industrial operation. In addition, the reaction can be carried out by a batch process, a semi-continuous process, a continuous process or the like. When a liquid containing the raw materials for reaction is passed through a fixed-bed, the catalyst is not contained at all or substantially in a liquid mixture discharged from a reaction zone.

In the epoxidation reaction in the present invention, when a fixed bed is used, a catalyst layer may be divided into multi-stages and fresh cumene hydroperoxide and propylene may be fed divisionally to each of catalyst layers divided, further a reaction product discharged from an outlet of each of catalyst layers may be recycled to an inlet of the catalyst layer.

This method is effective for preventing a run-away caused by heat of the reaction and for carrying out stably the reaction under high yield.

In the present invention, propylene fed to the epoxidation reaction is usually 1 to 30 times by mole, preferably 5 to 20 times by mole per mole of fresh cumene hydroperoxide to be supplied for epoxidation. To use an excess amount of propylene to fresh cumene hydroperoxide is effective for keeping a yield of produced propylene oxide at high yield.

In the present invention, when the amount by mole of propylene supplied in the epoxidation reaction exceeds to that of fresh cumene hydroperoxide supplied in the epoxidation reaction, propylene after the epoxidation reaction is recovered by distillation operations of several stages and then recycled to the epoxidation reactor, again.

The concentration of propylene to be supplied to the epoxidation reaction in the present invention is usually 70 % by weight or more, preferably 80 % by weight or more. As other impurities, it may contain components substantially inert to epoxidation such as propane, ethane, ethylene, methane, propylene oxide and the like.

In the present invention, it is essential that the concentration of the water in propylene supplied to the epoxidation reaction in the present invention is 2% by weight or less and preferably 1% by weight or less.

Water reacts with propylene oxide produced in the epoxidation reaction to form glycols, and therefore decreases a yield of propylene oxide. Further, usually, after completion of the epoxidation reaction, propylene is recovered from the reaction liquid and propylene oxide is separated. However, because the water in the reaction liquid is separated to propylene oxide side and an extra energy is additionally required for separation of the water from propylene oxide, it leads to disadvantage in economical. From the above viewpoints, it is necessary to control the water concentration within the range of the present invention. As a method of controlling the concentration of water, any method of a method of removing a part or all of the water by distillation, extraction or the like; a method of converting into another compound through a reaction; a method of reducing the water concentration using an absorber, a membrane which can coagulate water with a hydrophilic property or the like; and the like, may be adopted.

### EXAMPLE

### Examples 1 to 3

Propylene containing a water amount shown in Table 1 and cumene hydroperoxide were continuously passed through a fixed bed flow reactor in which 85 g of a titanium-containing silicon oxide catalyst was packed, at a ratio of 8 times by mole of propylene per 1 mole of cumene hydroperoxide fed. The conversion of cumene hydroperoxide was set to 99 % by adjusting a reaction pressure to 4.8 MPa and controlling an inlet temperature. In this case, the reaction temperature was 100°C. A yield of propylene oxide to propylene fed (PO selectivity) is shown in Table 1.

**Table 1**

| | Example 1 | Example 2 | Example 3 |
|---|---|---|---|
| Water content (% by weight) | 0 | 0.1 | 0.2 |
| PO selectivity (%) | 99.7 | 99.6 | 99.4 |

### Example 4

Propylene containing 1% by weight of water, 30 parts by weight of cumene hydroperoxide and 70 parts by weight of cumene are continuously passed through a fixed bed flow reactor in which 85 g of a titanium-containing silicon oxide catalyst is packed, in a ratio of 10 times by mole of propylene per 1 mole of cumene hydroperoxide fed. The conversion of cumene hydroperoxide is set to 99 % by controlling the inlet temperature. In this case, the reaction temperature will be 60°C and a yield of propylene oxide to propylene fed will be 95% or more.

### Comparative Example 1

When an epoxidation reaction is carried out in the same manner as in Example 4 except that the water concentration in propylene is 3% by weight, propylene glycol and oligomers of propylene glycol and propylene oxide will be produced and the yield of propylene oxide to propylene fed will decrease to about 90% . When propylene oxide is separated from the reaction liquid after the epoxidation reaction, an extra energy is required in a separation step of propylene oxide and water compared to Examples because water in the reaction liquid is separated to a propylene oxide side.

### INDUSTRIAL APPLICABILITY

As described above, according to the present invention, there can be provided a process for producing propylene oxide, having excellent characteristics in which the process converts propylene into propylene oxide using cumene hydroperoxide as an oxygen carrier, can be conducted under high yield, and further can reduce a load in a purification step of propylene oxide as a product.

## Claims

1. A process for producing propylene oxide, which comprises reacting propylene with cumene hydroperoxide in the presence of a catalyst, wherein a concentration of water contained in the propylene as a raw material is 2% by weight or less.

2. The process for producing propylene oxide according to claim 1, wherein the concentration of water contained in the propylene as a raw material is 1% by weight or less.
